# EUROPEAN PATENT APPLICATION

(11) **EP 1 598 039 A2**
(43) Date of publication of application: **23.11.2005**
(21) Application number: 05009464.8
(22) Date of filing: 29.04.2005
(51) Int. Cl.: A61F 13/74, A61F 13/56

(54) **Fastening members for sanitary napkins to undergarments and undergarments comprising said fastening members**

(30) Priority: 03.05.2004 US 567613 P
(71) Applicant: Tredegar Film Products Corporation, Richmond, VA 23225 (US)
(72) Inventor: Gottwald, John David, Richmond, VA 23233 (US); Donnelly, Constance Shanahan, Richmond, VA 23220 (US); Cree, James W., Chesterfield, VA 23838 (US); Smith, LaShara, Richmond, VA 23235 (US)
(74) Representative: Finck, Dieter

(57) **Abstract**

Securing members and articles for undergarments are provided. Hygienic articles may be received by the securing members or articles, and the securing members and articles may be removeably or permanently attached to an undergarment, which may be disposable or reusable.

## Description

### CROSS-REFERENCE TO RELATED APPLICATIONS

This application claims the benefit of the filing date from U.S. Serial No. 60/567,613, filed on May 3, 2004, by John D. Gottwald and Constance S. Donnelly, which disclosure is incorporated herein by reference.

### BACKGROUND

The embodiments relate generally to undergarments, particularly undergarments for use in combination with hygiene and other articles. The embodiments further relate to securing members and articles for maintaining the position of hygiene articles, e.g., absorbent articles, therapeutic articles, combined absorbent and therapeutic articles, etc. within undergarments.

Hygiene articles, and more particularly, absorbent articles for absorbing bodily fluids and other exudates, especially for use in connection with undergarments, are known. For example, feminine napkins, feminine pads, panty liners, incontinence inserts, and the like, have been used for absorbing and retaining bodily fluids. These absorbent articles are typically positioned adjacent or in proximity to a portion of a body for absorbing and retaining fluids, such as body exudates.

In order to ensure proper operation of these absorbent articles, such as to minimize leakage, different connection members have been used to maintain the proper position of the absorbent articles within, for example, undergarments. Such connection members include adhesives, clips, adjustable/positionable members (e.g., positionable flaps) and complimentary fastening members (e.g., hook and loop fasteners). These connection members have been used specifically for maintaining the position of absorbent articles within undergarments.

Heretofore, these connection members for maintaining the position of the absorbent articles often are difficult to use, for example, it may be difficult to properly position the absorbent article within the undergarment, including repositioning and/or replacing the absorbent article if necessary. The connection members also may not function adequately or may be uncomfortable to a user due to their design. For example, these members may not adequately secure the absorbent article to the undergarment or may contact a user's skin in an uncomfortable area. Additionally, these connection members may only maintain the position of the absorbent article within an undergarment for short periods of time. Further, the connection members may cause damage to undergarments depending on the type of undergarment and extent of use of the absorbent article.

Therapeutic articles may be used in various manners as well. For example, a heating and/or cooling type pad may be positioned adjacent or in proximity to a portion of a body for heating and/or cooling areas. Traditionally, these therapeutic articles are positioned through use of either gravity or external force, such as, for example simply holding the pad to an area, wrapping it beneath a bandage and the like.

Heretofore, these positioning techniques often are difficult to use, for example, it may be difficult to properly position and retain the therapeutic article on the body. The need to reheat and/or recool a therapeutic article may involve a complicated series of unwrappings and rewrappings as well - made more difficult if the patient to which the article is applied needs to perform the tasks. The positioning techniques also may not function adequately or may be uncomfortable to a user due to their design.

Therapeutic articles may be desired to be used with absorbent articles as well. In this event, the difficulties of the prior art, some of which are identified above, may be magnified.

### BRIEF DESCRIPTION OF THE DRAWINGS

Figure 1 illustrates a front plan view of an undergarment in accordance with an embodiment.

Figure 2 illustrates a perspective view of an undergarment in accordance with an embodiment.

Figure 3 illustrates a top plan view of a crotch portion of an undergarment having securing members in accordance with an embodiment.

Figure 4 illustrates atop plan view of a securing member in accordance with an embodiment.

Figure 5 illustrates a top plan view of a crotch portion of an undergarment having securing members in accordance with an embodiment with a hygiene article secured therewith.

Figure 6 illustrates a top plan view of a crotch portion of an undergarment having a securing member in accordance with another embodiment.

Figure 7 illustrates another embodiment.

Figure 8 illustrates another embodiment.

Figure 9 illustrates another embodiment.

Figure 10 illustrates another view of the embodiment of Figure 9.

Figure 10 illustrates another view of the embodiment of Figure 9.

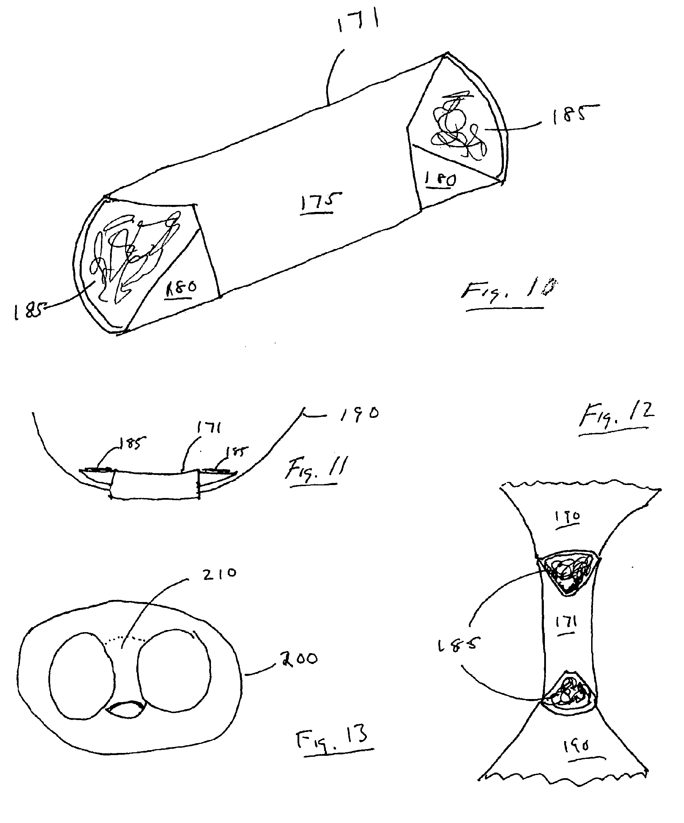

Figure 11 illustrates another view of the embodiment of Figure 9.

Figure 12 illustrates another view of the embodiment of Figure 9.

Figure 13 illustrates another embodiment.

### DETAILED DESCRIPTION OF THE EMBODIMENTS

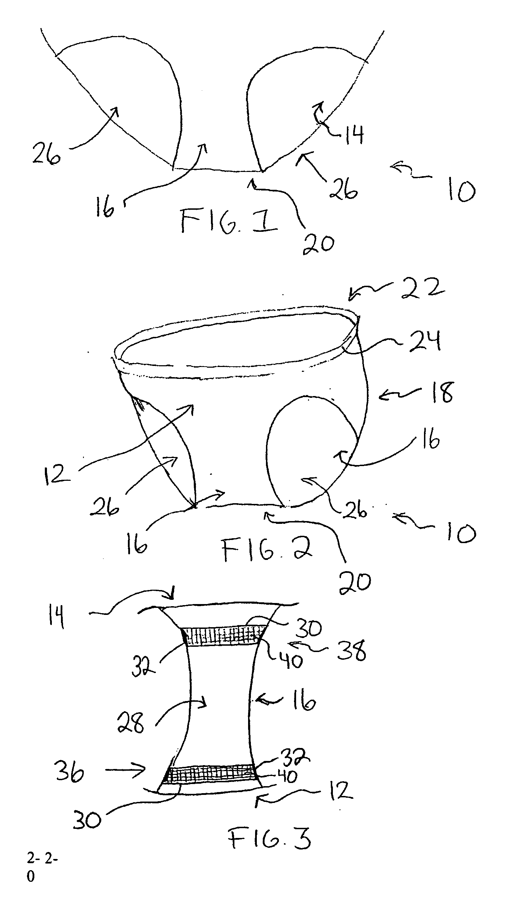

Figures 1 and 2 illustrate an undergarment 10, and more particularly, underwear in accordance with various embodiments. The undergarment 10 may be configured in different shapes and sizes for use by different types of individuals, including for example, by men, women, young people, old people, people with different medical conditions requiring use of a hygiene or incontinent article, etc. In general, the undergarment 10 includes a front portion 12, a back portion 14 and a crotch portion 16. The front portion 12, back portion 14 and crotch portion 16 together form a body portion 18 of the undergarment 10. The front portion 12, back portion 14 and crotch portion 16 may be constructed in a single unitary design or may be formed (e.g., seamed) from several component parts. The crotch portion 16 is located at a lower portion 20 of the undergarment 10 and generally extends between the front portion 12 and the back portion 14. An upper portion 22 of the undergarment 10 is defined by a waist portion 24, which may include, for example an elastic waist member (not shown). The front and back portions 12 and 14 each extend and are connected to the waist portion 24.

Openings 26 are defined by the front portion 12, back portion 14 and crotch portion 16, and more particularly, are provided at each side of the undergarment 10. The openings 26 allow a user's legs to pass therethrough for wearing the undergarment 10.

The undergarment 10 may be constructed of any suitable material, such as, for example, cotton or other material comfortable for wearing by a user. The material also may be configured to absorb or resist fluid depending on the use for the undergarment 10. For example, depending on the application, all or a portion of the undergarment 10 may be constructed of a hydrophilic or hydrophobic material. Additionally, the undergarment 10 may include separate linings (not shown) to provide additional support or fluid retaining/resisting properties as desired or needed.

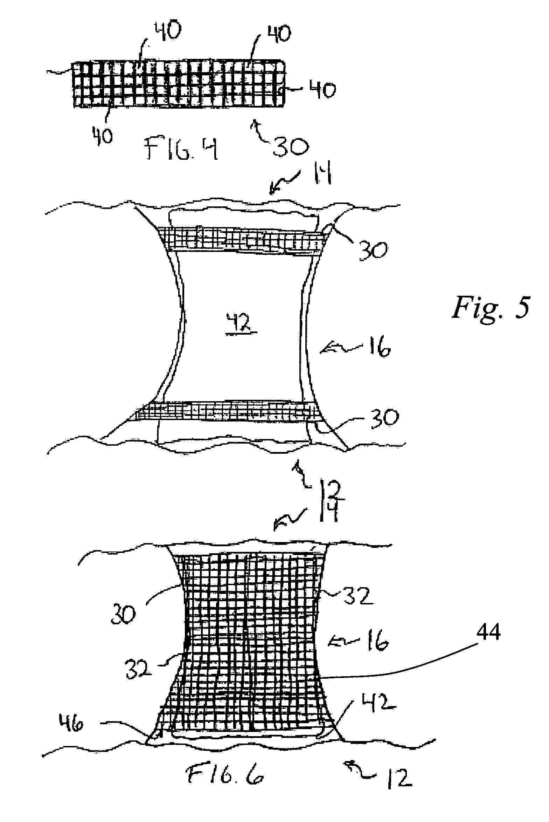

Figure 3 illustrates an inside portion 28 of the crotch portion 16 in accordance with an embodiment. The crotch portion 16 includes one or more securing members 30 attached to the crotch portion 16. The securing members 30 extend generally transversely across the crotch portion 16 and are secured to sides 32 of the crotch portion 16. For example, the securing members 30 may be securedly or permanently connected to the crotch portion 16 (e.g., sewn or seamed to the crotch portion 16). However, it should be noted that in various embodiments, the securing members 30 may be removably connected to the crotch portion 16, for example, using clip members or other suitable connection members. Further, although two securing members 30 are shown in Figure 3, additional or fewer securing members 30 may be provided as part of the undergarment 10 as desired or needed, for example, based on the size of the undergarment or type of hygiene article to be secured to the undergarment 10. For example, embodiments are described below that provide for application of a therapeutic article, such as heating pads, cooling pads, any other suitable pads, etc.

Various embodiments may be modified so as to provide for hygienic article application to a desired area or areas. So, for example, perineal treatment areas, anal treatment areas, tailbone treatment areas, genital area treatment areas, etc. may all result in modified hygienic article application areas. Embodiments may be sized as well for different sized individuals, for treatment areas, and for different garments.

A hygienic article may be retained internally in an undergarment or externally to an undergarment. Internal structures may require less retention, as the undergarment itself may serve to assist in holding an hygienic article in place.

Returning to Figure 3, the securing members 30 are constructed of an elastic material for expansion to secure and/or maintain thereunder a hygiene article. For example, the securing members 30 may include an elastomeric body portion for resisting movement of a hygiene article relative to the undergarment 10. In one embodiment, the securing members 30 are constructed of an elastic film and formed as straps that are secured to a front area 36 and back area 38 of the crotch potion 16. The elastic film may be formed partially of a plastic, composite or other suitable material.

The securing members 30 may be constructed of any elastomeric material and in one embodiment are formed having hydrophobic properties to promote fluid flow through apertures 40 as shown more clearly in Figure 4. For example, an elastic film for use in constructing the securing members 30 may be provided as described in U.S. Patent 5,733,628 entitled "Breathable Elastic Polymeric Film Laminates," which is incorporated by reference herein in its entirety. As used herein, the terms "elastic" and "elastomeric" mean a material which, upon application of a biasing force, is stretchable or extensible, to a stretched, biased length preferably at least 150% of its relaxed unbiased length, and that will retract at least 50% of its elongation upon release of the elongating force.

The securing members 30 may be connected to the crotch portion 16 such that more or less length is provided, for example, based on the type or size of hygiene article to be secured to the undergarment 10, such as, based on the height or thickness of the hygiene article. The securing members 30 may have a length substantially equal to the width of the crotch portion 16 across which the securing members 30 are attached, or may have a length longer or shorter that the width of that crotch portion 16.

As shown in Figure 4, the securing members 30 include a plurality of apertures 40 extending therethrough. As shown, the apertures 40 are configured having a square shape and extend generally along the entire length of the securing members 40 in a symmetric pattern or arrangement, for example, configured in a mesh arrangement. However, the apertures may be configured in any shape, including, for example, rectangles, triangles, circles, polygons, among others, and may be of the same or different sizes. Further, the apertures 40 may extend only along a portion of the securing members 30 and/or may be arranged in a non-symmetric or arbitrary pattern, for example, based on functional or aesthetic requirements. The apertures 40 may be formed in any manner known in the art, or hereafter developed, for making three-dimensional apertures, such as for example by vacuum forming, hydroforming, or hot pin aperturing.

Further, although the securing members 30 are shown connected to the front area 36 and back area 38 of the crotch potion 16, the positioning of the securing members 30 may be selected, for example, based on the type of undergarment 10, the anticipated user of the undergarment 10, the type of hygiene article to be secured, the application, etc. For example, one or more securing members 30 may be connected closer to the center or middle of the crotch portion 16 or higher along the front portion 12 or back portion 14.

Further, the size and shape of the securing members 30 may be modified as desired or needed, for example, based upon functional or aesthetic requirements. In one embodiment, the securing members 30 are configured having a generally rectangular shape with a length to allow connection to the sides 32 of the crotch portion 16 and a width of approximately one-half of an inch. In other embodiments, the width of the securing members 30 range between about one-eighth of an inch to two inches. It should be appreciated that the width of the securing members 30 may be modified to any dimension. Further, the thickness of the securing members 30 may be modified as desired or needed.

Various modifications to the embodiments are contemplated, such as, for example, providing a reinforced or lined inner crotch portion 16. For example, a hydrophilic material may be provided to all or part of the crotch portion 16 as desired or needed. Further, the securing members 30 may be formed or coated with a stain resistant material (e.g., plastic) and/or washable material. Additionally, the securing members 30 may be constructed of any type or kind of apertured and/or breathable elastomeric film or material.

Thus, as shown in Figure 5, the securing members 30 provide for resisting movement of a hygiene article 42 (e.g., a feminine hygiene pad or other absorbent article), for example, for securing and maintaining the position of the hygiene article 42 to the undergarment 10. Specifically, the securing members 30 may be expanded to insert a hygiene article 42 thereunder, and due to the elasticity of the securing members 30, the hygiene article 42 is maintained in the desired or required position and movement of the hygiene article 42 resisted. Further, if repositioning of the hygiene article 42 is desired or needed, a user may move the hygiene article 42 with the undergarment 10 removed or while wearing the undergarment 10.

It should be noted that the hygiene article 42 may be any type or kind of absorbing, retaining or protecting article for use in applications involving exudates or any type of bodily fluid or liquid. As used herein, the term "absorbing article" or "absorbent article" means articles that absorb and contain body exudates. More specifically, the term refers to articles that are placed against or in proximity to the body of a wearer for absorbing and containing various exudates discharged from the body, such as, for example, diapers, incontinent articles, sanitary napkins, pantiliners, bandages, and other suitable articles used to absorb body exudates.

In operation, the securing members 30 facilitate the retention of a hygiene article 42 to an undergarment 10 while the apertures 40 allow passage of exudates therethrough to the hygiene article 42 (e.g., absorbent article). The securing members 30 contact portions (e.g., ends) of the hygiene article 42 to resist movement of and maintain the position of the hygiene article 42 thereunder.

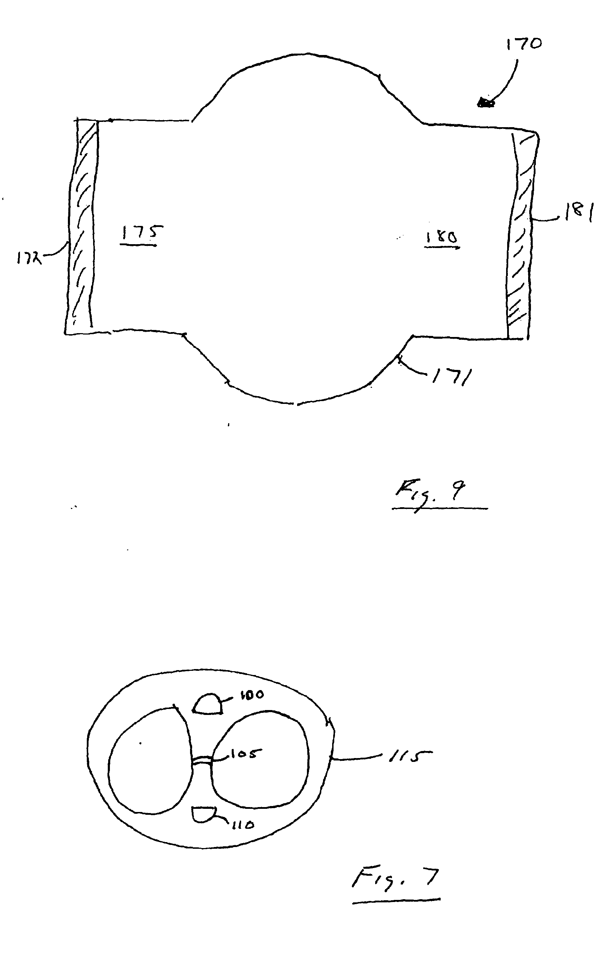

Although the securing members 30 are shown connected to the front area 36 and back area 38 of the crotch potion 16, the positioning of the securing members 30 may be modified, for example, to improve user comfort or retention of the hygiene article 42. Further, the securing members 30 may be modified and constructed to form different types of retention members. For example, as shown in Figure 6, a single securing member 30 may be configured to form a pocket 44, envelope or other receiving member for receiving the hygiene article 42 therein. In one embodiment, and as shown, the pocket 44 is formed by connecting the securing member 30 to the sides 32 of the crotch portion 16 and along the back area 38 of the crotch portion 16. The securing member 30 is not connected to the front area 36 of the crotch portion 16, thereby defming an opening 46 allowing insertion of the hygiene article 42. This embodiment provides contact of the securing member 30 along a substantial portion of the hygiene article 42.

Figure 7 shows another embodiment. Securing areas 100, 105 and 110 are used to secure a hygienic article in undergarment 115, which is shown in top view. In this embodiment, hygienic article (not shown) is a cooling pad type article, which provides therapeutic benefits to the applied area, and may be removed as desired. Preferred embodiments use a cooling pad known as Femé Pad™ manufactured by Florri-Femé Pharmaceuticals, Ltd. Leeds, England. Of course, heating or any other suitable therapeutic articles may be desirously used as well, in this and other embodiments. Moreover, various types of hygienic pads (absorbent, cooling, heating, etc.) may be interchanged as desired in various embodiments.

In Figure 7, securing areas 100 and 110 are a closed end (e.g., pocket - type) construction, sized to adequately secure the ends of the cooling pad. Although securing areas are a nonelastic construction in certain embodiments, and so sized to secure a desired hygienic article, other embodiments may utilize a elastic construction in addition to or instead of a nonelastic construction. If elastic construction is used, a securing area may stretch adequately for insertion of a hygienic article and then retract so as to hold the hygienic article once in place. As was described above, embodiments may provide for connecting elements, e.g., adhesive, hook and loop fasteners and/or other suitable means for retaining a hygienic article.

Returning to the embodiment of Figure 7, securing area 105 is of open ended construction, e.g., a strap, in the preferred embodiments of elastic, wherein strap 105 stretches adequately for insertion of the hygienic article while retracting so as to hold the hygienic article once in place. Of course, in various embodiments, as was described above, a securing area may be placed in various areas of an undergarment, so as to enable hygienic article retention.

It should also be noted that hygienic articles may be replaced as desired. For example, in those embodiments with a cooling pad such as a Femé Pad™, the article may be replaced on a regular schedule, so as to recool or insert a cooled article.

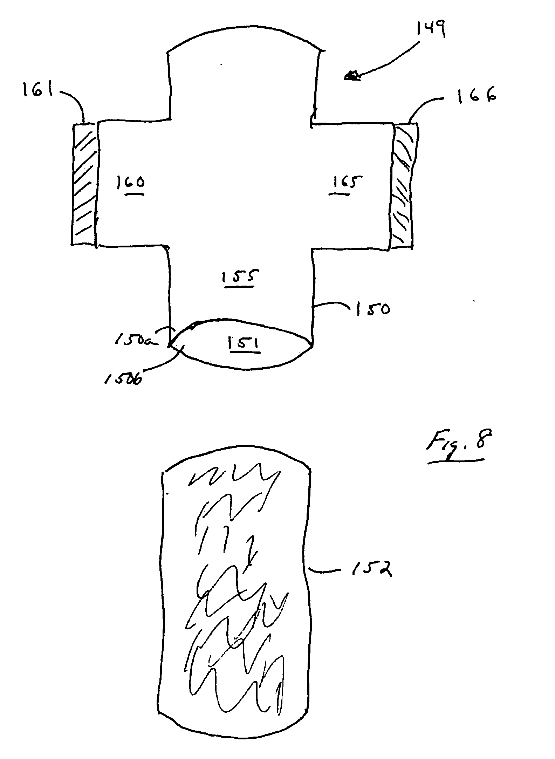

Figure 8 shows an embodiment generally at 149. Hygienic article sleeve 150 has body 155 and wings 160 and 165. Fastening areas 161 and 166, present on wings 160 and 165 respectively, are adhesive, although any suitable means may be used in various embodiments. This embodiment is a disposable embodiment, although it should be noted that other embodiments may be reused.

Hygienic article sleeve 150 has a recess within body 155 in preferred embodiments so as to provide for insertion and/or reinsertion of a therapeutic article. For example, opening 151 may receive a therapeutic article 152. Opening 151 may be simply left open, or be closed using any suitable means, such as for example, an overlapping flap, etc.

Hygienic article sleeve 150 has a topsheet 150a and backsheet 150b. Topsheet 150a is constructed of any material suited for the intended use. For example, if the sleeve 150 is to be worn on the interior of the undergarment 10, it would be preferred to make the topsheet 150a from a soft material, e.g., nonwoven, woven, etc. For additional comfort, one might desire that the topsheet 150a be a breathable material if it is to be worn in contact with or close proximity to the wearer's skin. Furthermore, it might be desired to have a topsheet, in this or other embodiments, be liquid permeable (such as an apertured formed film or nonwoven) to permit the passage of body fluids therethrough.

For example, an absorbent layer may be placed below a top sheet to absorb body exudates. In certain embodiments, the absorbent layer will comprise an absorbent pad.

Finally, if a film is used, it would be preferable for the film to be pliable and consideration should be given to the amount of noise that the film might generate in use.

On the other hand, if the sleeve 150 is to be worn on the exterior of the undergarment 10, the requirements of softness, breathable, and liquid permeability are not as important, but noise level would still be a consideration.

Similarly, backsheet 150b may be constructed of appropriate materials such as formed films, microporous breathable films, cast or blown liquid impervious films, nonwovens, wovens and the like, depending on its particular intended use and the desired properties such as softness, liquid permeability, breathability, noise level and pliability.

Suitable films and nonwovens for use in constructing the topsheet and/or backsheet are well known and widely used in commercially available absorbent article, such as diapers, adult incontinent products, panti liners and the like.

Backsheet 150b may also be provided with an adhesive coating (not shown) to facilitate attachment to an undergarment. Any suitable adhesive may be used in various embodiments. The adhesive coating may be applied to all of the backsheet 150b, or only to a portion thereof.

Embodiments may provide for absorbency, in a topsheet, backsheet and/or wings. Moreover, any suitable additional absorbent layers of materials may be added. Additionally, philic and/or phobic materials and/or treatments may be used to provide for additional liquid attraction and/or absorbency, in a topsheet, backsheet, wings and/or in additional layers.

Returning to Figure 8, either internal or external undergarment attachment may be provided. For example, an adhesive coating may be attached to an internal crotch area, wings 160 and 165 wrapped about the crotch area and fastening areas 161 and 166 fastened. Similar uses are provided for external wear, e.g., with wings 161 and 166 being wrapped below or about a crotch of any undergarment and fastened with their respective fasteners. As was described above, in a winged embodiment, the wings may provide for additional exudate control by being absorbent as well as hydrophilic and/or phobic.

Wings 160 and 165 may be constructed from the same type of films and nonwovens used to construct the topsheet 150a and/or backsheet 150b, depending on the same considerations of softness, breathability, pliability, noise level and liquid permeability. In various embodiments, wings might be provided with absorptive attributes, e.g., a core, etc, so they provide additional absorption for exudates and the like.

Figure 9 shows another embodiment. Here the hygienic article, shown generally at 170, comprises a single layer of nonwoven material 171. Fastening areas 172 and 181, present on wings 175 and 180 respectively, are adhesive, although any suitable means may be used in various embodiments. This embodiment is a disposable embodiment, although it should be noted that other embodiments may be reused.

Figure 10 shows the embodiment of Figure 9 folded about a hygienic article 185. In this embodiment, each wing 175 and 180 provide a securing means for the article 185. Of course, it may not be desired to have an embodiment with two wings, as a lighter weight with article security may also be achieved by an embodiment with a single wing.

Figures 11 and 12 show the embodiment of Figure 9, wrapped around the crotch of an undergarment 190. Each are shown in cutaway view, with Figure 11 being a side cutaway view of article 170, and Figure 12 being a top cutaway view.

Of course, in the embodiments shown in Figure 8, Figure 9 and other embodiments, wing size in relation to the sleeve and any hygienic article may be varied, as may the sleeve itself and any article held therein. In certain embodiments, only one wing with fastening area may be present, or wings may be dispensed with entirely. These embodiments preferably are for internal undergarment use, as they may use, as was described above, the undergarment itself to assist in article and/or sleeve retention. It also should be noted that the wings provide lateral support, thus, in other embodiments, straps, or any suitable means may be used to provide lateral support in addition to or instead of a wing or wings. Such lateral support may be provided through elastic, nonelastic or a combination of elastic and non-elastic lateral supports.

Figure 13 shows a top view of a disposable undergarment 200, which is provided with a sleeve 210 at least partially in the crotch portion for insertion of a hygienic article. In this embodiment, the sleeve may be made of the same material as the undergarment, that is, a breathable elastic laminate comprising an elastic core sandwiched between two nonwoven webs. Thus, a simple construction may be provided in this and other embodiments wherein a single topsheet is fastened to the edges of a crotch in order to reduce material that may be undesirable as it might add to thickness and discomfort. An elastic topsheet might be used as well, in order to assist in securely retaining a hygienic article. In this and other embodiments, absorbency may be provided as well, in the topsheet or in additional layers. Thus, a disposable undergarment such as that shown in Figure 13 may provide for both application of hygienic articles, e.g., cooling pads, warming pads, etc. while absorbing body exudates.

Embodiment may utilize adjustable securing areas to provide for retention of various size hygienic articles. Moreover, securing areas may be provided, in various embodiments, with enhancement mechanisms for hygienic articles. For example, embodiments may be provided with channels in securing or other areas to direct therapeutic effects of therapeutic articles, such as directed heating or cooling zones, etc. Article and/or undergarment thickness and/or materials could be similarly adapted.

In various embodiments, hygienic articles may be disposable, not disposable or partially disposable. For example, a cooling pad such as a Femé Pad™ may be reused in one or more disposable articles.

Various embodiments may facilitate the retention of a hygiene article to an undergarment. Embodiments may resist movement of a hygiene article and so secure and/or maintain the hygiene article within undergarments. Embodiments may be implemented in combination with single-use (e.g., disposable) and/or multiple-use (e.g., washable) undergarments. Further, embodiments may not be limited to use in connection with undergarments, but may be implemented in connection with any type of garment against which a hygiene article is to be maintained. Further, embodiments may be implemented in combination with hygiene articles having connection elements provided therewith, such as, for example, an adhesive portion.

Embodiments may be constructed as well of various materials depending upon use. For example, embodiments used in a commercial setting, such as a hospital, nursing home, other health care facility, etc. may be constructed of heavier and/or reusable material, etc. depending upon facility needs, user needs, etc.

While various specific embodiments have been described herein, those skilled in the art will recognize that the various embodiments can be practiced with modification within the spirit and scope of the claims.

## Claims

1. A securing member for an undergarment, said securing member comprising:
- an elastomeric body configured to connect to an undergarment and extend across a crotch portion of the undergarment; and
- a plurality of apertures formed within the elastomeric body.

2. The securing member of claim 1 wherein the elastomeric body is configured to receive a hygiene article thereunder.

3. The securing member of claim 1 wherein the elastomeric body is configured to connect to at least one of a front area and back area of the crotch portion of the undergarment.

4. The securing member of claim 1 wherein the elastomeric body comprises a stain resistant material.

5. The securing member of claim 1 wherein the elastomeric body is configured to removably connect to the crotch portion of the undergarment.

6. The securing member of claim 1 wherein the plurality of apertures are configured in a symmetric arrangement.

7. The securing member of claim 1 wherein the elastomeric body forms a pocket for receiving a hygiene article therein.

8. The securing member of claim 1 wherein the elastomeric body comprises a plastic film.

9. An undergarment comprising:
- a front portion;
- a back portion;
- a crotch portion extending between the front and back portion; and
- at least one securing member extending across the crotch portion, the at least one securing member comprising an elastic portion having a plurality of apertures formed therein.

10. The undergarment of claim 9 wherein the at least one securing member connects to each of a side of the crotch portion.

11. The undergarment of claim 9 wherein the at least one securing member is configured to have a length greater than a width of the crotch portion.

12. The undergarment of claim 9 wherein the at least one securing member is configured to form a pocket on the crotch portion.

13. The undergarment of claim 9 wherein at least one securing member is connected to a front area of the crotch portion and at least one securing member is connected to a back area of the crotch portion.

14. The undergarment of claim 9 wherein the at least one securing member comprises a mesh configured body.

15. The undergarment of claim 9 wherein the at least one securing member comprises a washable material.

16. A method of constructing an undergarment, said method comprising:
- forming a plurality of apertures in a securing member, the securing member having an elastic body; and
- attaching the securing member across a crotch portion of an undergarment.

17. The method of claim 16 wherein the attaching comprises removably connecting the securing member across the crotch portion.

18. A disposable undergarment comprising:
- a front portion;
- a back portion;
- a crotch portion extending between the front and back portion;
- a first closed end securing area;
- a second closed end securing area;
- a third open end securing area;
- wherein each of said first, second and third securing areas are sized to removeably secure a therapeutic article.

19. A disposable undergarment as in claim 18 wherein each of said first, second and third securing areas are sized to removeably secure a cooling pad therapeutic article.

20. A disposable undergarment as in claim 18 wherein each of said first, second and third securing areas are sized to removeably secure a heating pad therapeutic article.

21. A disposable article as in claim 18 where said third securing area is comprised of elastic.

22. An article for an undergarment comprising:
- a topsheet;
- a backsheet;
- a body having a recess for receiving therein a therapeutic article; and
- a wing with a fastening area.

23. An article as in claim 22 wherein said body having a recess for receiving therein a therapeutic article comprises a body having a recess for receiving therein a cooling pad therapeutic article.

24. An article as in claim 22 further comprising a second wing with a fastening area.

25. An article as in claim 22 wherein said topsheet is absorbent.

26. An article as in claim 22 further comprising an absorbent layer placed below said topsheet.

27. An article as in claim 22 wherein said backsheet further comprises an adhesive coating.

28. An undergarment comprising:
- a front portion;
- a back portion;
- a crotch portion extending between the front and back portion;
- a removable hygienic article sleeve, with a topsheet; a backsheet; a body having a recess for receiving therein a therapeutic article; and a wing with a fastening area.

29. A method for supplying a cooling pad to an undergarment comprising applying a hygienic article sleeve with a topsheet, a backsheet, a body having a recess for receiving therein a therapeutic article, and a wing with a fastening area; inserting a therapeutic article in said recess, and wrapping said wing about a crotch area of said undergarment.

30. An undergarment comprising;
- a front portion;
- a back portion;
- a crotch portion extending between the front and back portion;
- a sleeve located at least partially in said crotch portion for receiving a hygienic article.

31. A disposable undergarment as in claim 30 where said a sleeve located at least partially in said crotch portion for receiving a hygienic article further comprises a sleeve located at least partially in said crotch portion for receiving a therapeutic article.

32. A disposable undergarment as in claim 30 where said a sleeve located at least partially in said crotch portion for receiving a hygienic article further comprises a sleeve located at least partially in said crotch portion for receiving a cooling pad therapeutic article.
